(19) Europäisches Patentamt
European Patent Office
Office européen des brevets

(11) Publication number: 0 272 462 B1

(12) # EUROPEAN PATENT SPECIFICATION

(45) Date of publication of patent specification: **10.06.92** (51) Int. Cl.5: **C07J 41/00**, A61K 31/575

(21) Application number: **87117184.9**

(22) Date of filing: **21.11.87**

(54) **Process for preparing ursodeoxycholic acid derivates and their inorganic and organic salts having therpeutic activity.**

(30) Priority: **26.11.86 CH 4729/86**

(43) Date of publication of application:
**29.06.88 Bulletin 88/26**

(45) Publication of the grant of the patent:
**10.06.92 Bulletin 92/24**

(84) Designated Contracting States:
**AT BE CH DE ES FR GB GR IT LI LU NL SE**

(56) References cited:
**EP-A- 0 119 040**
**BE-A- 901 069**

**CHEMICAL ABSTRACTS, vol. 51, no. 22, 25th November 1957, page 17965, abstract no. 17965e-h, Columbus, Ohio, US; T. KANAZAWA et al.: "Synthesis of ursodeoxycholic acid", & NIPPON KAGAKU ZASSHI 76, 463-5(1955)**

**CHEMICAL ABSTRACTS, vol. 104, no. 7, 17th February 1986, page 539, abstract no. 51022g, Columbus, Ohio, US; & JP-A-60 161 996 (EISAI CO., LTD) 23-08-1985**

(73) Proprietor: **Jago Research AG**
**Seestrasse 65**
**CH-6052 Hergiswil NW(CH)**

(72) Inventor: **Reiner, Alberto**
**Via Mentana 23**
**Como(IT)**

(74) Representative: **Frei, Alexandra Sarah**
**Frei Patentanwaltsbüro Hedwigsteig 6 Postfach 95**
**CH-8029 Zürich(CH)**

EP 0 272 462 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The presente invention concerns a process for preparing amides of ursodeoxycholic acid of general formula:

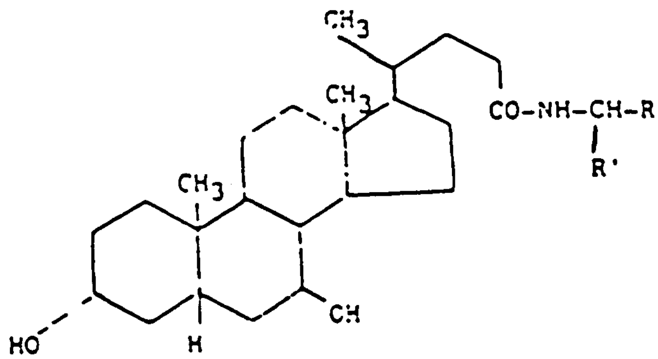

wherein:

R = $-CH_2-CH_2-SO_3H$ R' = -H

or:

R = -COOH

R' = $CH_2-CH_2-CONH_2$;$-CH_2-CONH_2$;$-CH_2-CH_2-SCH_3$; $-CH_2-S-CH_2-COOH$

as well as their organic salts with pharmaceutically active bases.

Said derivatives are therapeutically employed mainly in the treatment of altered biligenetic functions, lithiasis or diskenesya of biliary ducts.

The salts of the respective derivatives with aminoacids (lysine or arginine) or with inorganic ions (Na, Ca, etc.) are also to be considered.

The process for amide preparation provides for:

a) the preparation of the mixed anhydride of ursodeoxycholic acid with ethyl, phenyl, isobutyl chloroformate;

b) the reaction of said mixed anhydride with appropriate, reactive compounds, containing the $-NH_2$ group, whereby the amidic bond is formed.

Process 1° step

The anhydride of ursodeoxycholic acid is prepared while maintaining the temperature in the range between 6° and 20°C, using dioxane as solvente.

Isobutyl, phenyl or ethyl chloroformate and two moles of triethylamine are employed. The formation of anhydride takes about one hour under constant stirring.

Process II° step

The resulting mixed anhydride is reacted with the $-NH_2$ group containing reagent, at low temperature and in the presence of an organic amine. At reaction completed, appropriate treatments follow and the following derivatives are obtained:

a) when R = $-CH_2-CH_2-SO_3H$
   R' = -H TAURINAMIDE

b) when R = -COOH
   R' = $-(CH_2)_2-CONH_2$ GLUTAMINAMIDE

c) when R = -COOH
   R' = $-CH_2-CONH_2$ ASPARAGINAMIDE

d) when R = -COOH
   R' = $-CH_2-CH_2-S-CH_3$ METHIONINAMIDE

e) when R = -COOH
   R' = $-CH_2-S-CH_2-COOH$ CARBOXYMETHYL-CYSTEINAMIDE.

The process according to the present invention will be now explained with reference to the following

examples.

Example 1

Methioninamide of ursodeoxycholic acid
(R = -COOH; R' = $CH_2$-$CH_2$-S- $CH_3$)

a) Preparation of mixed anydride

To a suspension of 66,7 g of ursodeoxycholic acid in dioxane, 13,3 ml of ethyl chloroformate are added and the inner temperature of the system is adjusted to between 0° and 10°C.

The dioxane solution of the organic amine, preferably triethylamine, is slowly poured.

The heterogeneous, white material is then heated to room temperature.

b) Formation of amide

An aqueous solution of 27,9 g of a methionine salt with an organic amine is slowly added to the mixture resulting from a) and cooled to low temperature.

The heterogeneous, white material becomes more and more fluid, giving rise to a colourless solution when the addition is completed.

The solution temperature is allowed to increase spontaneously to 27-29°C over a course of about 5 hours, while an evolution of carbon dioxide takes place.

When the preparation of amide is completed, the product is separated by dilution with aqueous HCl and successively extracted by an organic solvent. After separation, the organic phase is treated with an aqueous solution of $NaHCO_3$ under stirring.

The two phase are separated and the aqueous solution is acidified, whereby white crystals are precipitated over a course of about 4 hours under vigorous stirring.

The resulting product, filtered under vacuum, is chromatographically pure, m.p. 107°C, with decomposition at 88-90°C.

The assay by quantitative, alkalimetric analysis is 98,51%, the impurity of free ursodeoxycholic acid being less than 0,3%. Melting point determination in a capillary tube presents a certain decomposition at 90°C, but the substance is perfectly clear a 107°C.

NMR and IR spectra prove its structural formula. No absorption is observed in the UV region, which happens also in the cases of compounds of the following Examples.

Example 2

Carboxymethyl-Cysteinamide of ursodeoxycholic acid
(R= -COOH; R' = -$CH_2$-S-$CH_2$-COOH)

The preparation follows very closely Example 1, with a sufficient amount of amine being employed to salify both the carboxy groups of carboxymethylcysteine.

The formation of amide from the mixed anhydride takes in about 2 hours and occurs at low temperature.

The amide formation is completed in about 6-8 hours.

With the procedure employed in Example 1, a product is obtained, in form of a microcrystalline, white powder, chromatographically pure, melting at 55°C;
The amount of free ursodeoxycholic acid, by quantitative alkalimetric analysis, is less then 0,5%.

NMR and IR spectra, prove its structural formula.

The melting point, determined in the capillary tube, is 55°C.

Example 3

Asparaginamide of ursodeoxychlic acid
(R = -COOH; R' = -$CH_2CONH_2$)

After the preparation of the mixed anhydride of ursodeoxycholic acid, the amide formation occurs always at low temperature by slowly adding the triethylamine salt.

In this case too the reaction is completed in about 6-8 hours.

Following the procedure of Example 1, after discharging and purification, a white crystalline product is obtained, which is chromatographically pure.

It melts at 117 ° C, with decomposition at 90 ° C.

The assay by quantitative, alkalimetric analysis is 99%, and the amount of free ursodeoxycholic acid is less than 0,5%.

NMR and IR spectra prove its structural formula.

A decomposition occurs at 90 ° C, but the substance is completely liquid in the capillary tube at 117 ° C.

Example 4

Glutaminamide of ursodeoxycholic acid
(R = -COOH; R′ = $-CH_2-CH_2-CONH_2$)

The reaction of triethylamine salt of glutamine with the mixed anhydride of ursodeoxycholic acid occurs, as for the previous reactions, at low temperature, without cooling bath, over a course of about 7 hours for completion.

The mixture is then discharged as in the Example 1. White crystals are obtained, chromatographically pure and melting at 110 ° C, with decomposition at 85 ° .

The amount of free ursodeoxycholic acid is less than 0,5%, by quantitative alkalimetric analysis.

NMR and IR spectra prove its structural formula. The product decomposes at 85 ° C and is completely liquid ad 110 ° C.

Example 5

Ca salt of taurinamide of ursodeoxycholic acid
(R = $-CH_2-CH_2-SO_3H$; R′ = -H)

In ths case too the reaction of the mixed anhydride of ursodeoxycholic acid is carried out by slowly adding the organic amine salt of taurine at low temperature.

After 5 hours under stirring the amide formation is completed and the reaction mixture is acidified with HCl.

The solution is then treated with $CaCO_3$.

No precipitation of the Ca salt is observed in the solution, which is then concentrated to dryness, collected in chloroform for removing the organic amine hydrochloride.

The chloroform phase is then decanted and the oily, thick, white substance is collected in acetone up to complete crystallization.

The resulting white crystals are chromatographically pure and have a melting point of 110 ° C;
The amount of free ursodeoxycholic acid is less than 0,5% by quantitative alkalimetric analysis.

NMR and IR spectra prove its structural formula.

**Claims**

**1.** Process for preparing Therapeutically active derivatives of ursodeoxycholic acid of general formula:

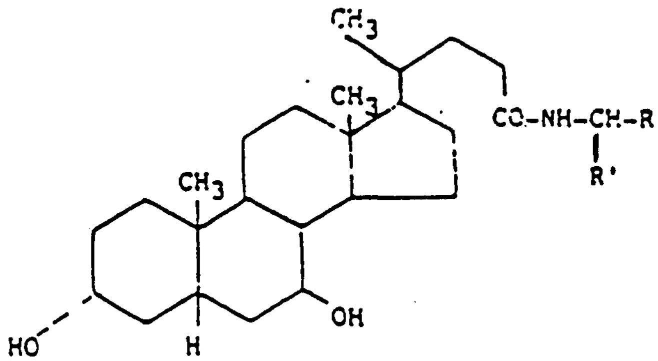

wherein R is radical selected from $-CH_2-CH_2-SO_3H$ and -COOH and R' is a radical selected from H and $-(CH)_2-CONH_2$ $-CH_2$ $-CONH_2$ , $-(CH_2)_2$ $-SCH_3$ , $-CH_2$ $-S-CH_2$ -COOH, respectively, and their organic and inorganic salts,

characterized by the steps wherein:

a) the mixed anhydride of ursodeoxycholic acid with an alkyl or phenyl chloroformate is formed, and

b) the above mixed anhydride is reacted with a reactive compound containing the $-NH_2$ group.

**2.** Process according to claim 1, characterized in that said chloroformate is selected from ethyl, phenyl and isobutyl chloroformate.

**3.** Process according to claim 1, characterized in that said step a) is carried out in the presence of an organic amine, at temperature not in excess of 20 ° C.

**4.** Process according to claim 1, characterized in that said reactive compounds are selected from organic amine salts of methionine, carboxymethylcysteine, asparagine, glutamine and taurine.

**5.** Process according to claim 3, characterized in that said organic amine is triethylamine.

**6.** Process according to claim 4, characterized in that said organic amine is triethylamine.

**7.** Process according to claim 1, characterized in that said step a) is carried out in dioxane as solvent.

**8.** Process according to claim 1, characterized in that said step b) is carried out at low temperature.

## Revendications

**1.** Procédé de préparation de dérivés d'acide ursodésoxycholique, thérapeutiquement actifs, représentés par la formule générale :

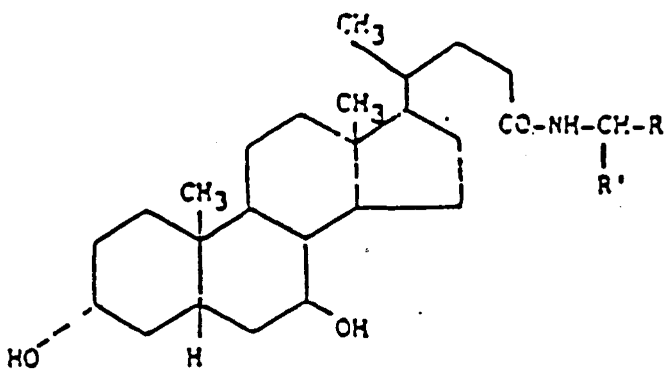

(I)

dans laquelle :

- R est un radical choisi parmi $-CH_2-SO_3H$ et -COOH ; et
- R' est un radical choisi parmi H et, respectivement, $-(CH_2)_2-CONH_2$, $-CH_2-CONH_2$, $-(CH_2)_2-SCH_3$, $-CH_2-S-CH_2-COOH$,

et leurs sels organiques et minéraux,

caractérisé par les étapes dans lesquelles :

(a) l'anhydride mixte d'acide ursodésoxycholique avec un chloroformiate d'alkyle ou de phényle est formé ; et

(b) l'anhydride mixte ci-dessus est mis à réagir avec un composé réactif contenant le groupe $-NH_2$.

**2.** Procédé selon la revendication 1, caractérisé par le fait que ledit chloroformiate est choisi parmi le chloroformiate d'éthyle, de phényle et d'isobutyle.

**3.** Procédé selon la revendication 1, caractérisé par le fait que ladite étape (a) est conduite en présence d'une amine organique, à une température ne dépassant pas 20 ° C.

4. Procédé selon la revendication 1, caractérisé par le fait que lesdits composés réactifs sont choisis parmi les sels d'amine organique de méthionine, carboxyméthylcystéine, asparagine, glutamine et taurine.

5. Procédé selon la revendication 3, caractérisé par le fait que ladite amine organique est la triéthylamine.

6. Procédé selon la revendication 4, caractérisé par le fait que ladite amine organique est la triéthylamine.

7. Procédé selon la revendication 1, caractérisé par le fait que ladite étape (a) est effectuée dans le dioxanne comme solvant.

8. Procédé selon la revendication 1, caractérisé par le fait que ladite étape (b) est effectuée à basse température.

## Patentansprüche

1. Verfahren zur Herstellung aktiver Derivate der Ursodesoxycholsäure mit der allgemeinen Strukturformel:

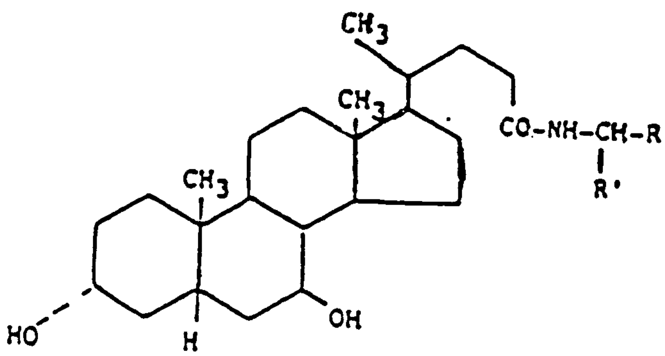

wobei R = $-CH_2-CH_2-SO_3H$ bzw. $-COOH$
und R' = $-H$ bzw. $-(CH_2)_2-CONH_2$, $-CH_2-CONH_2$, $-(CH_2)_2-SCH_3$,
oder $-CH_2-S-CH_2COOH$ ist und deren organische und anorganische Salze, **gekennzeichnet** durch die Verfahrensschritte
a) Aus Ursodesoxycholsäure und einem phenyl- oder alkylsubstituierten Formylchlorid wird ein gemischtes Anhydrid gebildet und
b) das gemischte Anhydrid wird mit einer reaktiven Verbindung, die eine $-NH_2$ Gruppe aufweist, zur Reaktion gebracht.

2. Verfahren nach Anspruch 1, **dadurch gekennzeichnet,** dass das substituierte Formylchlorid Ethyl-, Phenyl- oder Isobutyl-substituiert ist.

3. Verfahren nach Anspruch 1, **dadurch gekennzeichnet,** dass der erste Verfahrensschritt (a) in der Gegenwart eines organischen Amines und bei einer Temperatur, die nicht über 20 °C liegt, ausgeführt wird.

4. Verfahren nach Anspruch 1, **dadurch gekennzeichnet,** dass die reaktive Verbindung ein Salz eines organischen Amines und Methionin, Carboxymethyl-Cystein, Asparagin, Glutamin oder Taurin ist.

5. Verfahren nach Anspruch 3, **dadurch gekennzeichnet,** dass das organische Amine Triethylamin ist.

6. Verfahren nach Anspruch 4, **dadurch gekennzeichnet,** dass das organische Amin Triethylamin ist.

7. Verfahren nach Anspruch 1, **dadurch gekennzeichnet,** dass im ersten Verfahrensschritt (a) Dioxan als Lösungsmittel verwendet wird.

8. Verfahren nach Anspruch 1, **dadurch gekennzeichnet,** dass der zweite Verfahrensschritt (b) bei

niedriger Temperatur durchgeführt wird.